(19) Europäisches Patentamt
European Patent Office
Office européen des brevets

(11) Veröffentlichungsnummer: **0 089 922**
**B1**

(12) # EUROPÄISCHE PATENTSCHRIFT

(45) Veröffentlichungstag der Patentschrift: **27.07.88**

(51) Int. Cl.⁴: **C 08 K 5/42**

(21) Anmeldenummer: **83810096.4**

(22) Anmeldetag: **07.03.83**

(54) **Säurehärtbare Zusammensetzung enthaltend einen Maskierten Härtungskatalysator und Verfahren zu deren Härtung.**

(30) Priorität: **12.03.82 CH 1557/82**
**21.09.82 CH 5577/82**

(43) Veröffentlichungstag der Anmeldung: **28.09.83 Patentblatt 83/39**

(45) Bekanntmachung des Hinweises auf die Patenterteilung: **27.07.88 Patentblatt 88/30**

(84) Benannte Vertragsstaaten:
**BE DE FR GB IT NL SE**

(56) Entgegenhaltungen:
**EP-A- 0 037 152**
**DE-A- 1 919 678**
**DE-A- 2 616 414**
**FR-A- 2 125 962**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Kirchmayr, Rudolf, Dr.**
**Ettingerstrasse 9**
**CH-4147 Aesch (CH)**
Erfinder: **Rutsch, Werner, Dr.**
**Kleestrasse 7**
**CH-4153 Reinach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 089 922 B1

**Beschreibung**

Die Erfindung betrifft Zusammensetzungen auf Basis eines säurehärtbaren Harzes enthaltend einen maskierten Härtungskatalysator sowie ein Verfahren zur Härtung dieses Harzes durch Bestrahlung mit kurzwelligem Licht und anschliessendes Erwärmen.

Säurehärtbare Harze werden vor allem als Bindemittel für Lacke, Druckfarben und Anstrichfarben verwendet, wenn hohe Einbrenntemperaturen vermieden werden sollen. Säurehärtbare Harze können Aminoharze sein, einschliesslich verätherte, veresterte oder anderweitig modifizierte Melaminharze, Harnstoff-Formaldehydharze, Phenol-Formaldehydharze sowie Gemische solcher Harze mit Alkyd-, Polyester- oder Acrylharzen. Weitere säurehärtbare Harze sind Methylol-Verbindungen, Methyloläther von Polycarbonsäureimiden, z. B. Derivate von Polyacryl- oder Methacrylsäure, Urethanalkyde sowie Harze, die Carbonsäureester von N-Methylolimiden enthalten. Als saure Härtungskatalysatoren werden vorwiegend organische Säuren verwendet, darunter beispielsweise Sulfonsäuren, insbesondere p-Toluolsulfonsäure. Da diese Säuren bereits bei Raumtemperatur eine langsame Härtung bewirken, werden sie dem Harz erst kurz vor dessen Applikation zugesetzt, was mit den bekannten Problemen der Einhaltung bestimmter Topfzeiten verbunden ist. Zur Ermöglichung von Einkomponenten-Systemen hat man daher bereits die Verwendung maskierter Härtungskatalysatoren vorgeschlagen, aus denen bei erhöhter Temperatur die Säure freigesetzt wird. Beispiele hierfür sind Aminsalze von aromatischen Sulfonsäuren, wie die in der US-Patentschrift 3 474 054 vorgeschlagenen Pyridinsalze. Diese haben den Nachteil, dass sie bereits während der Lagerung eine langsame Härtung bewirken. Ausserdem entstehen dabei Geruchsprobleme.

Es wurde weiterhin vorgeschlagen, maskierte Härtungskatalysatoren zu verwenden, aus denen durch Bestrahlung mit UV-Licht der eigentliche Härtungskatalysator gebildet wird. Beispiele hierfür sind aromatische Sulfoniumsalze von komplexen Anionen, wie sie in der US-Patentschrift 4 102 687 beschrieben sind. Solche Sulfoniumsalze sind jedoch schwierig in reiner Form herzustellen, sie sind von geringer Reaktivität und neigen zur Vergilbung der Harze. Nach dem gleichen Prinzip wirkend wurden bereits photolabile Sulfonsäureester vorgeschlagen, so beispielsweise Sulfonsäureester des α-Hydroxymethylbenzoins, wie sie z. B. in der DE-OS 1 919 678 beschrieben sind. Diese Verbindungen genügen jedoch nicht in jeder Hinsicht den gestellten Anforderungen wie beispielsweise ausgezeichnete Lagerbeständigkeit, einwandfreie Löslichkeit in den säurehärtbaren Harzsystemen, geringere Vergilbung der Harze nach der Härtung sowie keine negative Beeinflussung des Lackierens nach dem elektrophoretischen Auftrageverfahren.

Es wurde nun gefunden, dass Sulfonsäureester bestimmter β-Hydroxycarbonylverbindungen, die technisch einfach herstellbar sind, diese Anforderungen erfüllen, indem sie im Dunkeln unbegrenzt lagerbar sind, bei Belichtung mit kurzwelligem Licht jedoch rasch zerfallen, was eine anschliessende säurekatalytische Härtung der Harze bei relativ niedriger Temperatur ermöglicht und nicht zur Vergilbung der Harze führt.

Gegenstand der Erfindung ist eine härtbare Zusammensetzung enthaltend ein säurehärtbares Harz und als maskierten Härtungskatalysator eine Verbindung der Formel I

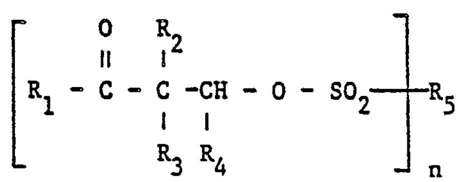

(I)

worin

n die Zahl 1 oder 2 ist,

$R_1$ ein unsubstituiertes oder durch 1, 2 oder 3 Reste —Cl, —Br, $C_1$-$C_8$ Alkyl, Phenyl, $C_1$-$C_8$-Alkoxy, Phenyloxy, Benzyloxy, $C_1$-$C_8$ Alkylthio, Phenylthio, $—SCH_2CH_2OH$, $C_1$-$C_4$ Alkyl-CONH—, Benzoylamino, Dimethylamino oder durch Benzoyl substituiertes Phenyl oder Naphthyl, oder $R_1$ ferner Anthryl oder Phenanthryl ist,

$R_2$ Wasserstoff, —OH, $C_1$-$C_4$ Alkoxy, $—OSi(CH_3)_3$, $—OCOCH_3$ oder unsubstituiertes oder durch Phenyl substituiertes $C_1$-$C_8$ Alkyl,

$R_3$ Wasserstoff, unsubstituiertes oder durch Phenyl substituiertes $C_1$-$C_8$ Alkyl, —CN, Benzoyl, $C_1$-$C_4$ Alkylcarbonyl oder $C_2$-$C_5$ Alkoxycarbonyl,

$R_4$ Wasserstoff, unsubstituiertes oder durch —OH, —Cl oder Phenyl substituiertes $C_1$-$C_8$ Alkyl, unsubstituiertes oder durch —OH, —Cl, $C_1$-$C_4$ Alkyl oder $C_1$-$C_4$ Alkoxy substituiertes Phenyl, $C_2$-$C_6$ Alkenyl, $C_8$-$C_9$ Phenylalkenyl, Furyl, Thienyl, $—CCl_3$, oder gesättigtes oder ungesättigtes $C_5$-$C_6$ Cycloalkyl bedeuten, oder ferner $R_1$ mit $R_3$, $R_3$ mit $R_4$ oder $R_2$ mit $R_3$ zusammen mit dem Kohlenstoffgerüst, an das sie gebunden sind, einen 5- oder 6-Ring bilden, welcher 1 bis 5 $—CH_2—$, $—CH(CH_3)—$, $—C(CH_3)_2—$, —O—, —S—, —SO—, $—SO_2—$, —CO—, $—N(CO-C_1-C_4\ Alkyl)—$ oder $—N(COC_6H_5)$-Gruppen enthält, und

$R_5$ wenn n = 1, $C_1$-$C_{18}$ Alkyl, unsubstituiertes oder durch —Cl, —OH, $C_1$-$C_{12}$ Alkyl, $C_1$-$C_4$ Alkoxy, $C_1$-$C_4$ Alkyl-CONH—, Benzoylamino, —$NO_2$ oder Benzoyl substituiertes Phenyl, unsubstituiertes oder durch —Cl, $C_1$-$C_{12}$ Alkyl oder $C_1$-$C_4$ Alkoxy substituiertes Naphthyl, oder $R_5$ ferner $C_5$-$C_6$ Cycloalkyl, $C_7$-$C_9$ Aralkyl, Campheryl, —$CF_3$, —$CCl_3$, —F oder —$NH_2$ bedeutet, und

$R_5$ wenn n = 2, eine —$(CH_2)_m$— Gruppe, wobei m die Zahl 2 bis 8 darstellt, oder unsubstituiertes oder durch $C_1$-$C_{12}$ Alkyl substituiertes Phenylen oder Naphthylen ist.

Sind Phenyl, Naphthyl als $R_1$ durch $C_1$-$C_8$ Alkyl substituiert, so handelt es sich um geradkettige oder verzweigte Substituenten, wie beispielsweise Methyl, Aethyl, n-Propyl, Isopropyl, n-Butyl, sec.-Butyl, tert.-Butyl, Pentyl, Hexyl, Heptyl, Octyl oder 2-Aethylhexyl, insbesondere um Methyl, Aethyl, n-Propyl, Isopropyl, n-Butyl oder tert.-Butyl, bevorzugt um Methyl. Sind Phenyl, Naphthyl als $R_1$ durch $C_1$-$C_8$ Alkoxy substituiert, so handelt es sich beispielsweise um Methoxy, Aethoxy, Propoxy, Isopropoxy, tert.-Butoxy, Pentyloxy oder Octyloxy, insbesondere aber um Methoxy, Aethoxy, n-Propoxy und n-Butoxy. Sind Phenyl, Naphthyl als $R_1$ durch $C_1$-$C_8$ Alkylthio substituiert, so handelt es sich um geradkettige oder verzweigte Substituenten, wie beispielsweise Methyl-, Aethyl-, n-Propyl, Isopropyl-, n-Butyl-, sec.-Butyl-, tert.-Butyl-, Pentyl-, Hexyl-, Heptyl- oder Octyl-, insbesondere aber um Methylthio.

Als $C_1$-$C_8$ Alkyl sind $R_2$, $R_3$ und $R_4$ geradkettige oder verzweigte Alkylgruppen, vorzugsweise aber geradkettige $C_1$-$C_4$ Alkylgruppen, wie beispielsweise Methyl, Aethyl, n-Propyl oder n-Butyl.

Stellt $R_2$ $C_1$-$C_4$ Alkoxy dar, so handelt es sich beispielsweise um Methoxy, Aethoxy, n-Propoxy, Isopropoxy, Butoxy oder tert.-Butoxy Substituenten.

Stellen $R_2$, $R_3$ und $R_4$ durch Phenyl substituiertes $C_1$-$C_8$ Alkyl dar, so handelt es sich beispielsweise um Benzyl oder Phenyläthyl Gruppen.

Stellt $R_3$ $C_1$-$C_4$ Alkylcarbonyl dar, so handelt es sich beispielsweise um Methyl-, Aethyl-, Propyl- oder tert.-Butylcarbonyl Substituenten.

Ist $R_3$ $C_2$-$C_5$ Alkoxycarbonyl, so handelt es sich beispielsweise um Methoxy-, Aethoxy-, Isopropoxy-, Butoxy- oder tert.-Butoxycarbonyl Substituenten.

Ist Phenyl als $R_4$ durch $C_1$-$C_4$ Alkyl oder $C_1$-$C_4$ Alkoxy substituiert, so handelt es sich um Methyl, Aethyl, n-Propyl, Isopropyl, n-Butyl oder tert.-Butyl resp. um Methoxy, Aethoxy, n-Propoxy, Isopropoxy, n-Butoxy oder tert.-Butoxy Substituenten.

Stellt $R_4$ $C_2$-$C_6$ Alkenyl dar, so handelt es sich beispielsweise um Vinyl, 1-Propenyl, 2-Propenyl, Isopropenyl, 2-Butenyl, Isobutenyl, 2-Pentenyl, 2-Hexenyl oder 5-Hexenyl Substituenten, insbesondere aber um Vinyl, Isobutenyl oder 1-Propenyl.

Stellt $R_4$ $C_8$-$C_9$ Phenylalkenyl dar, so handelt es sich um Styryl oder 3-Phenylpropenyl Substituenten, insbesondere um Styryl.

Stellt $R_4$ Furyl oder Thienyl dar, so kommen alle Stellungsisomeren in Betracht. Bevorzugte Stellungsisomeren sind jedoch 2-Furyl und 2-Thienyl.

Stellt $R_4$ ungesättigtes $C_5$-$C_6$ Cycloalkyl dar, so handelt es sich beispielsweise um 2-Cyclopenten-1-yl, 1-Cyclohexen-1-yl oder 3-Cyclohexen-1-yl Substituenten.

Wenn n = 1 :

Stellt $R_5$ $C_1$-$C_{18}$ Alkyl dar, so handelt es sich um geradkettige oder verzweigte Gruppen, wie beispielsweise Methyl, Aethyl, Propyl, Isopropyl, Butyl, tert.-Butyl, Pentyl, Hexyl, Heptyl, Octyl, Nonyl, Decyl, 2-Aethylhexyl, Undecyl, Dodecyl, tert.-Dodecyl, Tridecyl, Tetradecyl, Hexadecyl oder Octadecyl.

Ist Phenyl oder Naphthyl als $R_5$ durch $C_1$-$C_{12}$ Alkyl substituiert, so handelt es sich um geradkettige oder verzweigte Alkylgruppen, wie beispielsweise Methyl, Aethyl, Propyl, Butyl, tert.-Butyl, Pentyl, Hexyl, Octyl, Nonyl, Decyl oder Dodecyl.

Ist Phenyl oder Naphthyl als $R_5$ durch $C_1$-$C_4$ Alkoxy substituiert, so handelt es sich um Methoxy, Aethoxy, Propoxy, Isopropoxy, Butoxy oder tert.-Butoxy Substituenten.

Bedeutet $R_5$ Campheryl, so handelt es sich um 10-Campheryl.

Wenn n = 2 :

Stellt $R_5$ eine -$(CH_2)_m$- Gruppe dar, so handelt es sich beispielsweise um Aethylen, Propylen, Butylen, Pentylen oder Hexamethylen.

Bevorzugt sind Härtungskatalysatoren der Formel I, worin n 1 oder 2 ist,

$R_1$ ein unsubstituiertes oder durch -Cl, $C_1$-$C_8$ Alkyl, Phenyl, $C_1$-$C_4$ Alkoxy, Phenyloxy, $C_1$-$C_4$ Alkylthio, oder durch -$SCH_2CH_2OH$ substituiertes Phenyl oder Naphthyl ist,

$R_2$ Wasserstoff, -OH oder $C_1$-$C_8$ Alkyl,

$R_3$ Wasserstoff oder $C_1$-$C_8$ Alkyl, und

$R_4$ Wasserstoff, $C_1$-$C_4$ Alkyl, Phenyl, $C_2$-$C_6$ Alkenyl, Furyl oder -$CCl_3$ bedeuten, oder ferner $R_2$ mit $R_3$ oder $R_3$ mit $R_4$ zusammen mit den Kohlenstoffatomen, an die sie gebunden sind, einen Cyclohexylring bilden, und

$R_5$ wenn n = 1, $C_1$-$C_{18}$ Alkyl, unsubstituiertes oder durch -Cl, $C_1$-$C_{12}$ Alkyl oder $C_1$-$C_4$ Alkoxy substituiertes Phenyl, unsubstituiertes oder durch $C_1$-$C_{12}$ Alkyl substituiertes Naphthyl, oder $R_5$ ferner Campheryl, -$CF_3$ oder -F bedeutet, und

$R_5$ wenn n = 2, eine -$(CH_2)_m$-Gruppe, Phenylen oder Naphthylen ist, worin m die Zahl 2, 3 oder 4 darstellt.

Besonders bevorzugt sind Härtungskatalysatoren der Formel I, worin n die Zahl 1 ist, $R_1$ ein unsubstituiertes oder durch -Cl, $C_1$-$C_4$ Alkyl, $C_1$-$C_4$ Alkoxy, -$SCH_3$ oder Phenyl substituiertes Phenyl ist,

$R_2$ -OH oder $C_1$-$C_4$ Alkyl, $R^3$ $C_1$-$C_4$ Alkyl und $R_4$ Wasserstoff, $C_1$-$C_4$ Alkyl, Furyl oder $-CCl_3$ bedeuten, oder ferner $R_3$ mit $R_4$ zusammen mit den Kohlenstoffatomen, an die sie gebunden sind, einen Cyclohexylring bilden, und

$R_5$ $C_1$-$C_{18}$ Alkyl, unsubstituiertes oder durch $C_1$-$C_{12}$ Alkyl substituiertes Phenyl oder Naphthyl, oder Campheryl bedeutet.

Beispiel für einzelne Verbindungen der Formel I sind :

3-[(p-Tolylsulfonyl)oxy]-2-hydroxy-2-methyl-1-phenyl-1-propanon
3-[(Methylsulfonyl)oxy]-2-hydroxy-2-methyl-1-phenyl-1-propanon
3-[(Hexadecylsulfonyl)oxy]-2-hydroxy-2-methyl-1-phenyl-1-propanon
3-[(p-Chlorphenylsulfonyl)oxy]-2-hydroxy-2-methyl-1-phenyl-1-propanon
3-[(p-Laurylphenylsulfonyl)oxy]-2-hydroxy-2-methyl-1-phenyl-1-propanon
3-[(p-Aethoxyphenylsulfonyl)oxy]-2-hydroxy-2-methyl-1-phenyl-1-propanon
3-[(Phenylsulfonyl)oxy]-2-methyl-2-trimethylsiloxy-1-phenyl-1-propanon
3-[(β-Naphthylsulfonyl)oxy]-2-hydroxy-2-methyl-1-phenyl-1-propanon
1-Hydroxy-1-benzoyl-2-(p-tolylsulfonyl)oxy-cyclohexan
3-[(10'-Camphery lsulfonyl)oxy]-2-hydroxy-2-methyl-1-phenyl-1-propanon
3-[(p-Tolylsulfonyl)oxy]-2-hydroxy-2-methyl-1,3-diphenyl-1-propanon
3-[(Trifluormethylsulfonyl)oxy]-2-hydroxy-2-methyl-1,3-diphenyl-1-propanon
3-[(β-Naphthylsulfonyl)oxy]-2-hydroxy-2-methyl-1,3-diphenyl-1-propanon
3-[(p-Tolylsulfonyl)oxy]-2-hydroxy-2-methyl-1-phenyl-1-butanon
3-[(p-Tolylsulfonyl)oxy]-3-(2'-furyl)-2-hydroxy-2-methyl-1-phenyl-1-propanon
3-[(Phenylsulfonyl)oxy]-2-äthoxy-2-methyl-1,3-diphenyl-1-propanon
3-[(p-Tolylsulfonyl)oxy]-2-acetoxy-2-methyl-1-propanon
3-[(p-Tolylsulfonyl)oxy]-2-hydroxy-2-methyl-1-phenyl-4,4,4-trichlor-1-butanon
3-[(p-Tolylsulfonyl)oxy]-2,5-dihydroxy-2-methyl-1-phenyl-1-hexanon
3-[(p-Tolylsulfonyl)oxy]-2-hydroxy-2-methyl-1-phenyl-4-penten-1-on
3-[(Phenylsulfonyl)oxy]-1,5-diphenyl-2-hydroxy-2-methyl-1-phenyl-4-penten-1-on
3-[(10'-Camphery lsulfonyl)oxy]-3-(3"-cyclohexen-1"-yl)-2-hydroxy-2-methyl-1-phenyl-1-propanon
1-Benzoyl-1-[(p-tolylsulfonyl)oxy]methyl-cyclohexan
3-[(p-Tolylsulfonyl)oxy]-2,2-dimethyl-1-phenyl-1-propanon
3-[(p-Tolylsulfonyl)oxy]-3-(p-chlor)phenyl-2-hydroxy-2-methyl-1-phenyl-1-propanon
3-[(α-Naphthylsulfonyl)oxy]-2-hydroxy-3-(o-hydroxy)phenyl-2-methyl-1-phenyl-1-propanon
3-[(p-Tolylsulfonyl)oxy]-2-hydroxy-2-methyl-3-(p-methoxy)phenyl-1-phenyl-1-propanon
3-[(p-Tolylsulfonyl)oxy]-2-hydroxy-2-methyl-1-(p-chlor)phenyl-1-propanon
3-[(p-Tolylsulfonyl)oxy]-2-hydroxy-2-methyl-1-(p-methyl)phenyl-1-propanon
3-[(p-Tolylsulfonyl)oxy]-1,3-bis(p-methyl)phenyl-2-hydroxy-2-methyl-1-propanon
3-[(β-Naphthylsulfonyl)oxy]-2-hydroxy-2-methyl-1-(p-methylthio)phenyl-1-propanon
3-[(p-Tolylsulfonyl)oxy]-2-hydroxy-2-methyl-3-phenyl-1-p(β-hydroxy-äthylthio)phenyl-1-propanon
3-[(p-Tolylsulfonyl)oxy]-2-hydroxy-3-methyl-1-(β-naphthyl)-1-propanon
3-[(p-Phenylsulfonyl)oxy]-2-hydroxy-2-methyl-3-phenyl-1-(p-methoxy)-phenyl-1-propanon
3-[(p-Tolylsulfonyl)oxy]-2-hydroxy-2-methyl-1-(p-phenyloxy)phenyl-1-propanon
3-[(Methylsulfonyl)oxy]-2-hydroxy-2-methyl-1-(p-benzoyl)phenyl-1-propanon
3-[(p-Tolylsulfonyl)oxy]-2-äthoxycarbonyl-2-hydroxy-1-phenyl-1-propanon
3-[(p-Tolylsulfonyl)oxy]-2-benzoyl-2-hydroxy-1-phenyl-1-propanon
3-[(p-Tolylsulfonyl)oxy]-2-cyano-1,3-diphenyl-2-trimethylsiloxy-1-propanon
3-[(p-Tolylsulfonyl)oxy]-2-acetyl-2-hydroxy-1-phenyl-1-propanon
2-[[(p-Tolylsulfonyl)oxy]methyl]-2-hydroxy-tetralon-(1)
2-[[(β-Naphthylsulfonyl)oxy]methyl]-2-hydroxy-4-methyl-tetralon-(1)
3-[[(p-Tolylsulfonyl)oxy]methyl]-3-hydroxy-chromon
3-[[(p-Tolylsulfonyl)oxy]methyl]-3-hydroxy-1-thiochromanon-(4)
3-[[(10'-Camphery lsulfonyl)oxy]methyl]-3-hydroxy-1-thiochromanon-(4)-S-oxid
3-[[(p-Tolylsulfonyl)oxy]methyl]-1-acetyl-3-hydroxy-chinolon-(4)
2-[(p-Tolylsulfonyl)oxy]-3-hydroxy-1-thiochromanon-(4)-S-dioxid
2-[[(p-Tolylsulfonyl)oxy]methyl]-2-hydroxy-indanon-(1)
2-[α[(p-Tolylsulfonyl)oxy]-benzyl]-3,3-dimethyl-2-hydroxy-indanon-(1)
2-[[(p-Tolylsulfonyl)oxy]methyl]-3(2H)-benzofuran
3-[(p-Tolylsulfonyl)oxy]-2-hydroxy-2-methyl-indanon-(1)
2-[[(p-Tolylsulfonyl)oxy]methyl]-2-trimethylsiloxy-benzo[b]-thiophen-3(2H)-on.

Beispiele für einzelne Verbindungen der Formel I, worin n = 2 ist, sind :

Hexan-1,6-disulfonsäure-bis-(2'-benzoyl-2'-hydroxy)-propylester
Benzoldisulfonsäure-bis-[2'-benzoyl-1'-(2"-furyl)-2'-hydroxy]-propyl-ester
Dinonylnaphthalindisulfonsäure-bis-(2'-benzoyl-2'-hydroxy)-propyl-ester.

Mehrere Verbindungen der Formel I sind bekannt und können nach bekannten Verfahren hergestellt werden, beispielsweise durch Umsetzung der entsprechenden Epoxy-Verbindung der Formel II

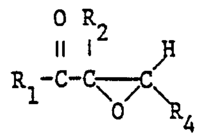

(II)

mit einem Aequivalent des entsprechenden Monosulfonsäure-Derivates der Formel III oder mit einem halben Aequivalent des Disulfonsäurederivats der Formel IV

$R_5SO_3H$ (III) $HO_3S\text{-}R_5\text{-}SO_3H$ (IV)

wie beispielsweise nach den in Ber. Deutsch. Chem. Ges. 69, 2753 (1936) oder in J. Chem. Soc., 1949, 315, beschriebenen Verfahren, oder durch Umsetzung einer entsprechenden Hydroxyverbindung der Formel V

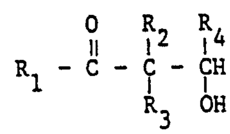

(V)

mit einem Mono- bzw. Disulfonsäurechlorid der Formeln $R_5\text{-}SO_2Cl$ bzw. $ClO_2S\text{-}R_5\text{-}SO_2Cl$ gemäss dem in der DE-OS 1 919 678 angegebenen Verfahren. In den Formeln II, III, IV und V haben die Reste $R_1$, $R_2$, $R_3$, $R_4$ und $R_5$ die oben angegebene Bedeutung.

Die benötigten Epoxyverbindungen der Formel II können nach an sich bekannten Verfahren hergestellt werden, so beispielsweise durch Chlorierung der entsprechenden Verbindung der Formel VI

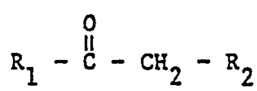

(VI)

zu dem entsprechenden Chloridderivat der Formel VII

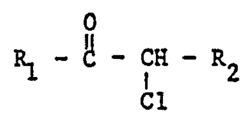

(VII)

welches durch anschliessende Kondensation mit dem entsprechenden Aldehyd der Formel VIII

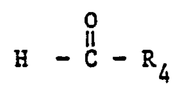

(VIII)

zu der entsprechenden Epoxyverbindung der Formel II umgesetzt wird [siehe diesbezüglich Chem. Soc. 75, 2042 (1953)],
oder aber durch eine Aldol-Kondensation der entsprechenden Verbindung der Formel VI mit dem entsprechenden Aldehyd der Formel VIII zu der entsprechenden Verbindung der Formel IX

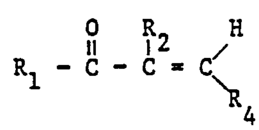

(IX)

welche anschliessend beispielsweise mittels Wasserstoffperoxid zu der entsprechenden Epoxy-Verbindung der Formel II umgesetzt wird [siehe diesbezüglich J. Chem. Soc., 79, 928 (1901) und Org. Syntheses 60, 88 (1981), für die Aldolkondensation, und J. Org. Chem. 28, 250 (1963) oder Org. Syntheses 55, 52 (1976) für die Bildung der Epoxyderivate].

In den Formeln VI, VII, VIII und IX haben die Reste $R_1$, $R_2$ und $R_4$ die oben angegebene Bedeutung.

Die Verbindungen der Formeln III und IV können nach bekannten Verfahren hergestellt werden, wie beispielsweise nach denjenigen, die in Houben-Weyl, Methoden der organischen Chemie, Band IX, S. 347 bzw. 435 beschrieben sind.

Andere Verbindungen der Formel I sind neu und stellen daher auch einen Gegenstand der vorliegenden Erfindung dar. Die Herstellung erfolgt in Analogie zu den bekannten Verbindungen.

Die neuen Verbindungen der Formel I entsprechen der Formel X

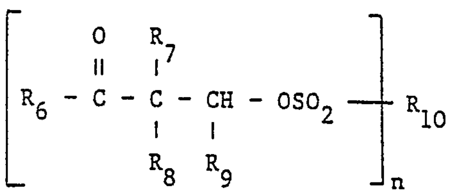

(X)

worin n die Zahl 1 oder 2 ist,

$R_6$ ein unsubstituiertes oder durch 1, 2 oder 3 Reste -Cl, -Br, $C_1$-$C_8$ Alkyl, Phenyl, $C_1$-$C_8$ Alkoxy, Phenyloxy, Benzyloxy, $C_1$-$C_8$ Alkylthio, Phenylthio, $-SCH_2CH_2OH$, $C_1$-$C_4$ Alkyl-CONH-, Benzoylamino, Dimethylamino oder Benzoyl substituiertes Phenyl oder Naphthyl, oder $R_6$ ferner Anthryl oder Phenanthryl ist,

$R_7$ -H, -OH, $C_1$-$C_4$ Alkoxy, $-OSi(CH_3)_3$, $-OCOCH_3$, $C_2$-$C_8$ Alkyl oder durch Phenyl substituiertes $C_1$-$C_8$ Alkyl,

$R_8$ unsubstituiertes oder durch Phenyl substituiertes $C_1$-$C_8$ Alkyl, -CN, Benzoyl, $C_1$-$C_4$ Alkylcarbonyl oder $C_2$-$C_5$ Alkoxycarbonyl, und

$R_9$ -H, unsubstituiertes oder durch -OH, -Cl oder Phenyl substituiertes $C_1$-$C_8$ Alkyl, unsubstituiertes oder durch -OH, -Cl, $C_1$-$C_4$ Alkyl oder $C_1$-$C_4$ Alkoxy substituiertes Phenyl, $C_2$-$C_6$ Alkenyl, $C_8$-$C_9$ Phenylalkenyl, Furyl, Thienyl, $-CCl_3$, oder gesättigtes oder ungesättigtes $C_5$-$C_6$ Cycloalkyl bedeuten, oder ferner $R_6$ mit $R_8$, $R_8$ mit $R_9$ oder $R_7$ mit $R_8$ zusammen mit dem Kohlenstoffgerüst, an das sie gebunden sind, einen 5- oder 6-Ring bilden, welcher 1 bis 5 $-CH_2-$, $-CH(CH_3)-$, $-C(CH_3)_2-$, -O-, -S-, $-SO_2-$, -CO-, -N(CO-$C_1$-$C_4$ Alkyl)-, oder $-N(COC_6H_5)$-Gruppen enthält, und

$R_{10}$ wenn n = 1, $C_1$-$C_{18}$ Alkyl, unsubstituiertes oder durch -Cl, -OH, $C_1$-$C_{12}$ Alkyl, $C_1$-$C_4$ Alkoxy, $C_1$-$C_4$ Alkyl-CONH-, Benzoylamino, $-NO_2$ oder durch Benzoyl substituiertes Phenyl, unsubstituiertes oder durch -Cl, $C_1$-$C_{12}$ Alkyl oder $C_1$-$C_4$ Alkoxy substituiertes Naphthyl, oder $R_{10}$ ferner $C_5$-$C_6$ Cycloalkyl, $C_7$-$C_9$ Aralkyl, Campheryl, $-CF_3$, $-CCl_3$, -F oder $-NH_2$ bedeutet, und

$R_{10}$ wenn n = 2, eine $-(CH_2)_m$-Gruppe oder durch $C_1$-$C_{12}$ Alkyl substituiertes Phenylen oder Naphthylen ist.

Mehrere Zwischenprodukte der Formeln II, III, IV, V, VI, VII, VIII und IX stellen bekannte Verbindungen dar, andere Zwischenprodukte der Formeln II bis IX sind neu. Die Herstellung erfolgt in Analogie zu den bekannten Verbindungen.

Die Härtungskatalysatoren der Formel I werden den Harzen in einer für die Härtung ausreichenden Menge zugesetzt. Die benötigte Menge hängt nicht nur von der Art des Harzes sondern auch von der beabsichtigten Härtungstemperatur und Härtungszeit ab. Im allgemeinen verwendet man 0,1 bis 10 Gew.%, vorzugsweise 0,5 bis 5 Gew.%, bezogen auf das lösungsmittelfreie Harz. Mischungen von Härtungskatalysatoren der Formel I können auch eingesetzt werden.

Als säurehärtbare Harze kommen alle Harze in Frage, deren Härtung durch saure Katalysatoren beschleunigt werden kann. Das sind vor allem Lacke auf Basis von Acryl-, Polyester-, Alkyd-, Melamin-, Harnstoff- und Phenolharzen, insbesondere aber die Mischungen von Acryl-, Polyester- oder Alkydharzen untereinander oder mit einem Melaminharz. Darunter fallen auch modifizierte Lackharze wie z. B. acrylmodifizierte Polyester- oder Alkydharze. Beispiele für einzelne Typen von Harzen, die unter den Begriff Acryl-, Polyester- und Alkydharze fallen, sind z. B. in Wagner, Sarx/Lackkunstharze (München, 1971), Seiten 86 bis 123 und 229 bis 238, oder in Ullmann/Encyclopädie der techn. Chemie, 4. Auflage, Band 15 (1978), Seiten 613 bis 628, beschrieben. Von besonderer Bedeutung ist die saure Katalyse für die Härtung von Lacken, die verätherte Aminoharze enthalten, wie z. B. methylierte oder butylierte Melaminharze (N-Methoxymethyl- bzw. N-Butoxymethylmelamin oder methylierte/butylierte Glycolurile usw.), z. B. :

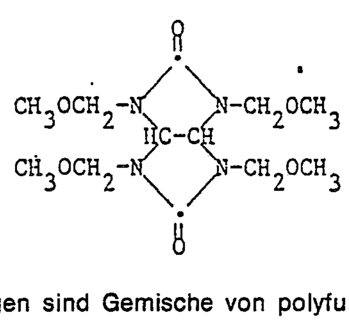

Weitere Harzzusammensetzungen sind Gemische von polyfunktionellen Alkoholen, oder Hydroxylgruppen enthaltenden Acryl- oder Polyesterharzen, oder partiell verseiftem Polyvinylacetat oder Polyvinylalkohol mit polyfunktionellen Dihydropyranyläthern, wie beispielsweise Derivate der 3,4-Dihydro-2H-pyran-2-carbonsäure.

Für bestimmte Zwecke verwendet man auch Harzzusammensetzungen, die monomere oder oligomere Bestandteile mit polymerisationsfähigen ungesättigten Gruppen haben. Auch solche Harzzusammensetzungen sind nach dem erfindungsgemässen Verfahren härtbar. Hierbei können zusätzlich radikalische

Polymerisationsinitiatoren oder Photoinitiatoren, beispielsweise aus der Klasse der aromatischen Ketone, Benzoinverbindungen, Benzylketale, α-Hydroxyacetophenonderivate oder die Verbindungen der Formel I mitverwendet werden. Erstere initiieren die Polymerisation der ungesättigten Gruppen während der Wärmebehandlung, letztere während der UV-Bestrahlung. Man kann solche Harzzusammensetzungen mit ungesättigten Komponenten auch durch Elektronenstrahlen polymerisieren. Zusätzlich zur Polymerisation der ungesättigten Komponenten muss aber stets eine sauer katalysierte Vernetzung (eventuell beim Einbrennen) erfolgen.

Die Lacke können Lösungen oder Dispersionen des Lackharzes in einem organischen Lösungsmittel oder in Wasser sein, sie können aber auch lösungsmittelfrei sein. Von besonderem Interesse sind Lacke mit geringem Lösungsmittelanteil, sogenannte « high solids-Lacke ». Die Lacke können Klarlacke sein, wie sie z. B. in der Automobilindustrie als Decklacke von Mehrschichten-Anstrichen verwendet werden. Sie können auch Pigmente enthalten, seien es anorganische oder organische Pigmente, sowie Metallpulver für Metalleffekt-Lacke.

Die Lacke können weiterhin kleinere Mengen an speziellen Zusätzen enthalten, wie sie in der Lacktechnologie üblich sind, beispielsweise Verlaufhilfsmittel, Thixotropiemittel, Lichtschutzmittel, Antioxydantien oder Photoinitiatoren.

Beispiele für Lichtschutzmittel sind solche aus der Klasse der Hydroxyphenyl-benzotriazole, Hydroxybenzophenone, Cyanacrylate, Hydroxyphenyltriazine, Oxalanilide, organischen Nickelverbindungen oder der Polyalkylpiperidinderivate. Da Lichtschutzmittel vom UV-Absorber-Typ die erfindungsgëmasse UV-Bestrahlung stören können, kann man solche Lichtschutzmittel auch einer angrenzenden Lackschicht zusetzen, aus der sie dann allmählich in die zu schützende Schicht des Einbrennlackes eindiffundieren. Die angrenzende Lackschicht kann eine Grundierung unter dem Einbrennlack oder ein Decklack über dem Einbrennlack sein.

Eine weitere Möglichkeit, den störenden Einfluss vom UV-Absorber zu umgehen, besteht in der Verwendung von sogenannten « geblockten UV-Absorbern », wie sie z. B. in der DE-OS 26 48 367 beschrieben sind. Ebenfalls geeignet sind Produkte, die unter Photo-Fries-Umlagerung UV-Absorber bilden wie z. B. Resorcinmonobenzoat oder gewisse Salicylsäureester.

Bevorzugt werden Polymethylpiperidinderivate bzw. Kombinationen derselben mit (geblockten) UV-Absorbern verwendet.

Die folgenden erfindungsgemässen Zusammensetzungen stellen spezielle Vorzugsformen der Erfindung dar:

a) Zusammensetzung, welche als säurehärtbares Hars ein Aminoharz oder ein Gemisch eines Aminoharzes mit einem anderen säurehärtbaren Harz enthält.

b) Zusammensetzung, welche als säurehärtbares Harz ein Phenolharz oder ein Gemisch eines solchen Harzes mit einem anderen säurehärtbaren Harz enthält.

c) Zusammensetzung, welche als Harz ein Gemisch aus mindestens einer polymerisierbaren Verbindung mit einer oder mehreren polymerisierbaren, äthylenisch ungesättigten Bindungen, und mindestens einem.

Aminoplast, wie ein Melamin- oder ein Harnstoffaldehydharz, und zusätzlich radikalische Polymerisationsinitiatoren und gegebenenfalls Photoinitiatoren enthält.

Beispiele für polymerisierbare Verbindungen mit einer oder mehreren polymerisierbaren äthylenisch ungesättigten Bindungen sind Ester der Acryl- und Methacrylsäure, Hydroxyäthylester der Acryl- und Methacrylsäure, Di- und Polyacrylate sowie Di- und Polymethacrylate von Glykolen und Polyolen, aromatische Vinyl- und Divinylderivate, N-Methylolderivate des Acrylamids oder Methacrylamids, Vinylalkyläther, Trimethylolpropandiallyläthermono-(meth)-acrylate, Reaktionsprodukte von Glycidyl(meth)acrylat und Mono- oder Dicarbonsäuren, Polyesterharze aus α,β-ungesättigten Dicarbonsäuren oder deren Anhydriden und Diolen, Urethanacrylate oder Polyepoxy-polyacrylate.

Bevorzugt sind dabei Zusammensetzungen aus

A) 80-99 Gew.% einer polymerisierbaren Verbindung mit einer oder mehreren äthylenisch ungesättigten Bindungen,

B) 1 bis 20 Gew.% mindestens eines Aminoplastes, wie Melamin- oder Harnstofformaldehydharz, und

C) 0,1 bis 10 Gew.%, bezogen auf die Summe aus A und B, eines Härtungskatalysators der Formel I.

Die Erfindung betrifft weiterhin ein Verfahren zur Härtung von säurehärtbaren Harzen in Gegenwart von Härtungskatalysatoren der Formel I durch Bestrahlen mit kurzwelligem Licht und anschliessendes Erwärmen.

Die Bestrahlung des Harzes mit kurzwelligem Licht geschieht vorzugsweise mit UV-Licht, wofür es heute eine Anzahl geeigneter technischer Geräte gibt. Diese enthalten Quecksilber-mitteldruck-, -hochdruck oder -niederdrucklampen sowie Leuchtstoffröhren, deren Emissionsmaxima bei 250 bis 400 nm liegen. Die nötigen Bestrahlungszeiten hängen von der Schichtdicke des Harzes, von der Pigmentierung, von der Lichtstärke der Lampen und von der Distanz der Lampen ab. Ein unpigmentierter Lack in üblicher Schichtdicke benötigt in üblichen UV-Bestrahlungsgeräten einige Sekunden Belichtungszeit. In dieser Zeit hat sich der latente Katalysator unter Bildung von freier Sulfonsäure photochemisch umgewandelt.

Setzt man dem Harz Photosensibilisatoren zu, so kann man die Bestrahlung auch mit Tageslichtlampen durchführen. Beispiele für bekannte Photosensibilisatoren sind kondensierte Aromaten wie z. B. Perylen, aromatische Amine (wie sie z. B. im US-Patent 4,069,054 beschrieben sind) oder kationische und basische Farbstoffe (wie sie z. B. im US-Patent 4,026,705 beschrieben sind).

Da die Säurehärtung bei Raumtemperatur sehr langsam abläuft, ist es für eine technische Ausübung des Verfahrens nötig, an die Bestrahlung eine Temperaturbehandlung anzuschliessen. Diese kann jedoch im Unterschied zu anderen Verfahren mit wärmespaltbaren Härtungskatalysatoren bei relativ niedrigen Temperaturen durchgeführt werden. Bei einer Einbrenndauer von etwa 30 Minuten und Verwendung von etwa 2 % Katalysator genügen Einbrenntemperaturen von etwa 70 bis 80 °C. Bei Verwendung von 1 % Katalysator benötigt man Temperaturen von etwa 80 bis 100 °C und bei Verwendung von 0,5 % Katalysator etwa 100 bis 120 °C. Vorzugsweise härtet man die erfindungsgemäss katalysierten Harze nach der Bestrahlung bei Temperaturen unterhalb 130 °C. Demgegenüber benötigt man für die Härtung mit bekannten Aminsalzen von Sulfonsäuren (ohne Bestrahlung) Einbrenntemperaturen von über 130 °C.

Diese relativ niedrigen Einbrenntemperaturen des erfindungsgemässen Verfahrens sind von erheblicher technischer Bedeutung bei der Beschichtung oder Lackierung von temperaturempfindlichen Substraten. Beispiele hierfür sind Gegenstände aus Holz oder Karton, insbesondere aber Gegenstände, die Teile aus Kunststoffe, oder Kautschuken enthalten, beispielsweise elektrische Geräte, Fahrzeuge aller Art oder Maschinen.

Ein weiterer Vorteil gegenüber anderen Einkomponenten-Harzen, die einen Härtungskatalysator enthalten, ist, dass die erfindungsgemässen Einkomponentensysteme bei Raumtemperatur praktisch unbegrenzt lagerfähig sind, da sich der wirksame Katalysator erst bei der Bestrahlung bildet.

Das erfindungsgemässe Verfahren ist für alle Arten der industriellen Beschichtung und Lackierung geeignet, wie z. B. für die Lackierung von Maschinen, Fahrzeugen, Schiffen oder Konstruktionsteilen. Von besonderer Bedeutung ist es für die Automobil-Lackierung. Hierbei kann es sowohl in Einschicht-Lackierung wie Mehrschicht-Lackierung angewendet werden. Von besonderem Interesse ist auch die Anwendung des Verfahrens für die kontinuierliche Beschichtung von Blechen, beispielsweise Stahl- oder Aluminiumblechen, nach dem sogenannten Coil-Coat-Verfahren. Das Verfahren eignet sich zudem für die Härtung von säurehärtbaren Druckfarben, die sich wegen ihrer hervorragenden Aufziehfähigkeit besonders für den Blechdruck eignen.

Bei der Anwendung des erfindungsgemässen Verfahrens auf Pressmassen, Giess- und Laminierharzen, können die Harze zuerst in dünner Schicht bestrahlt und anschliessend zu beliebigen Gegenständen heiss verformt und gehärtet werden. Soweit es sich jedoch um Gegenstände von relativ niedriger Dicke handelt, können die Harze auch zuerst verformt und anschliessend bestrahlt und erwärmt werden. Die Schichtdicke kann bei der Bestrahlung der Harze, je nach deren Transparenz, mehrere Millimeter betragen. Eine weitere Anwendungsmöglichkeit findet das Verfahren bei der Herstellung von Relief-Formen, wie z. B. Druckplatten. Hierbei erfolgt zunächst eine Belichtung der festen oder flüssigen säurehärtbaren Harzzusammensetzung, die auch ungesättigte Monomere/Präpolymere enthalten kann, durch einen Negativfilm. Anschliessend erfolgt gegebenenfalls eine thermische Nachbehandlung, wobei die belichteten Stellen vernetzt werden. Zum Schluss wird die Druckplatte durch Auswaschen der unvernetzten Teile entwickelt.

Die folgenden Beispiele erläutern das Verfahren anhand spezifischer erfindungsgemässer Zusammensetzungen näher. Hierin bedeuten Teile Gewichtsteile und % Gewichtsprozente. Der Druck wird in Bar bzw. Millibar angegeben.

## Herstellungsbeispiele

### Beispiel 1

In einem 1,5 Liter-Sulfierkolben, versehen mit Rührer, Thermometer und Stickstoffüberleitung, werden 126 Teile (0,75 Mol) α-Chlorpropiophenon, 24 Teile (0,8 Mol) Paraformaldehyd in 500 ml tert.-Butanol suspendiert, dann innerhalb einer Stunde portionsweise 95 Teile (0,85 Mol) festes Kalium-tert.-butylat zugegeben. Die Temperatur steigt dabei auf ca. 40 °C und es entsteht eine rotbraune Suspension, die noch 5 Stunden nachgerührt wird. Das Reaktionsgemisch wird dann auf 1 Liter Eis/Wasser gegossen und die so erhaltene Lösung zweimal mit je 750 ml Diaethyläther extrahiert. Die vereinigten organischen Lösungen werden mit $Na_2SO_4$ getrocknet, klarfiltriert und am Rotationsverdampfer vom Aether befreit. Nach Destillation unter Vakuum des roten Rückstands werden 85 Teile des Epoxids der Formel

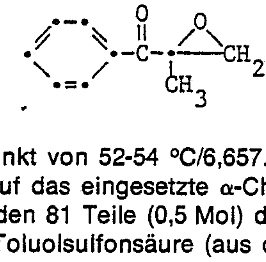

als gelbliches Oel mit einem Siedepunkt von 52-54 °C/6,657.$10^{-2}$ mBar erhalten. Dies entspricht einer Ausbeute von 70 % d. Th., bezogen auf das eingesetzte α-Chlorpropiophenon.

In einem 1 Liter-Rundkolben werden 81 Teile (0,5 Mol) der oben beschriebenen Epoxidverbindung und 86 Teile (0,5 Mol) wasserfreie p-Toluolsulfonsäure (aus dem entsprechenden Monohydrat azeotropisch entwässert, gemäss Houben-Weyl, Methoden der Organischen Chemie, Bd. 9, S. 436) in 500 ml Toluol während 16 Stunden unter Wasserausschluss bei 60 °C gerührt, wobei das Ende der Reaktion durch Dünnschichtchromatographie festgestellt wird. Die Reaktionslösung wird aud 1 Lit. einer eiskalten wässrigen Natriumbicarbonat-Lösung gegossen. Nach Abtrennung von der wässrigen Phase wird die organische Phase mit $Na_2SO_4$ getrocknet und am Rotationsverdampfer bei einer Badtemperatur von 40-50 °C unter Wasserstrahlvakuum eingeengt. Nach Umkritallisation des Rückstands aus 400 ml Diisopropyläther werden 100 Teile (60 % d. Th.) 3-[(p-Tolylsulfonyloxy)]-2-hydroxy-2-methyl-1-phenyl-1-propanon der Formel

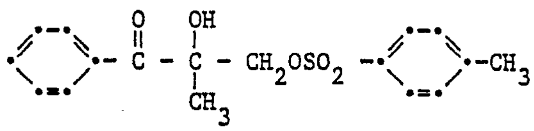

als weisses Pulver mit Schmelzpunkt 88-90 °C erhalten.

Verbrennungsanalyse :

| | | | |
|---|---|---|---|
| berechnet für $C_{17}H_{18}O_5S$ : | C 61,06 % | H 5,43 % | S 9,59 % |
| gefunden : | C 61,21 % | H 5,52 % | S 9,38 %. |

$H^1$-NMR ($CDCl_3$, δ in ppm bez. TMS) : 1,52 (S,3H) ; 2,42 (S,3H) ; 3,82 (S,1H) ; 4,13 und 4,43 (AB-System, J = 10 Hz, 2H) ; 7,0-8,0 (Multiplett, 9H). [S = Singulett, TMS = Trimethylsilan].

Die weiteren Beispiele 2-9 in der folgenden Tabelle 1 illustrieren weitere β-Sulfonyloxyverbindungen, die in analoger Weise wie nach Beispiel 1 hergestellt wurden.

(Siehe Tabelle 1 Seite 10 ff.)

Tableau 1 (Fortsetzung)

| Beispiel Nr. | Formel | Smp. °C | Molekular-Formel | Analyse (gefunden bzw. berechnet, in %) C | H | S |
|---|---|---|---|---|---|---|
| 2 | 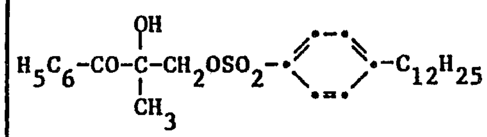 | Oel | $C_{28}H_{40}O_5S$ | 68,99<br>68,82 | 8,18<br>8,25 | -<br>- |
| 3 | 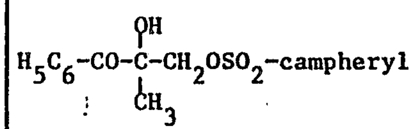 | 86-87 | $C_{20}H_{26}O_6S$ | 60,85<br>60,89 | 6,52<br>6,64 | 7,90<br>8,13 |
| 4 | 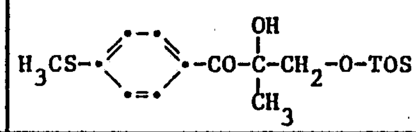 | 78-80 | $C_{18}H_{20}O_5S_2$ | 57,16<br>56,82 | 5,34<br>5,30 | 16,55<br>16,85 |
| 5 | 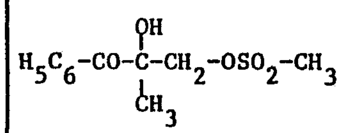<br>(mit Restlösungsmittelmenge) | Oel | $C_{11}H_{14}O_5S$ | 51,77<br>51,15 | 5,68<br>5,46 | 11,64<br>12,41 |
| 6 | 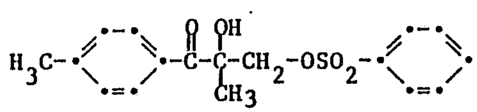<br>(mit Restlösungsmittelmenge) | Oel | $C_{11}H_{18}O_5S$ | 62,05<br>61,06 | 5,51<br>5,43 | 8,92<br>9,59 |

Tableau 1 (Fortsetzung)

| Beispiel Nr. | Formel | Smp. °C | Molekular-Formel | Analyse ( gefunden bzw. berechnet, in % ) | | |
|---|---|---|---|---|---|---|
| | | | | C | H | S |
| 7 | 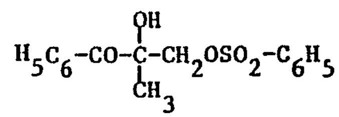 (mit Restlösungsmittelmenge) | Oel | $C_{16}H_{16}O_5S$ | 60,56<br>59,99 | 5,36<br>5,04 | 9,32<br>10,01 |
| 8 | 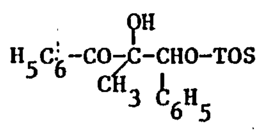 | 97-98 | $C_{23}H_{22}O_5S$ | 67,46<br>67,30 | 5,50<br>5,40 | 7,69<br>7,81 |
| 9 | 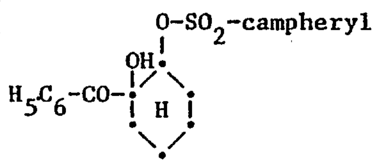 | 154-159 | $C_{23}H_{30}O_6S$ | 63,58<br>63,57 | 7,00<br>6,96 | 7,10<br>7,38 |
| 10 | 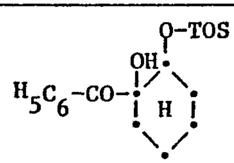 | 90-95 | $C_{20}H_{22}O_5S$ | 64,46<br>64,15 | 5,95<br>5,92 | -<br>- |

TOS = p-Tolylsulfonyl.

Anwendungsbeispiele

Beispiel II

Härtung eines Lackes auf Basis von Acryl-Melamin-Harz

Aluminiumbleche von 0,5 mm Dieke, die mit einem weisspigmentierten Grundlack auf Basis Polyesterharz beschichtet sind, werden mit einem festkörperreichen Klarlack der folgenden Zusammensetzung beschichtet:

| | | Festkörper |
|---|---|---|
| Hexamethoxymethylmelamin (Cymel 301, 100 %) | 17,93 g | 17,93 Teile |
| Butylacetat | 9,73 g | |
| Celluloseacetoburyrat (CAB 551001 der Fa. Eastman Chem.) | 1,83 g | |
| Silikonharz in organischem Lösungsmittel (Verlaufshilfsmittel Byketol Spezial der Fa. ByK-Mallinckrodt) | 2,80 g | |
| Verlaufshilfsmittel auf Polymer-Basis (Modaflow , 1 % ige Lsg., Monsanto) | 0,29 g | |
| Hydroxylfunktionelles Acrylharz (Paraloid AT 410, 73 Gew.% ; Rohm + Haas) | 57,30 | 41,83 Teile |
| n-Butanol | 10,12 g | |
| | 100,00 g | 59,76 Teile |

Der Katalysator wird in einer Menge von 1 %, bezogen auf lösungsmittelfreies Bindemittel (59,76 Teile), in einem Teil des Butanols vorgelöst und in die obige Harzformulierung eingearbeitet.

Der Lack wird mit einem elektrischen Filmziehgerät so aufgetragen, dass die Trockenfilmstärke etwa 30 μ beträgt. Nach einer Abluftzeit von 15 Minuten werden die Proben in einem PPG-Bestrahlungsgerät mit 2 Hochdruckquecksilberdampflampe à 80 Watt einer UV-Bestrahlung unter Variation der Bestrahlungszeit ausgesetzt. Anschliessend werden die Proben in einem Lackofen 30 Minuten bei 100 °C eingebrannt.

Zur Beurteilung des Härtungsgrades wird die Pendelhärte des Lackfilms nach der Methode von König (DIN 53 158) bestimmet, und zwar 30 Minuten und 24 Stunden nach dem Einbrennen.

Zur Beurteilung der Verfärbung (Vergilbung) wird der Farbtonabstand Δ E gemäss DIN 6174 bestimmt.

Die Resultate sind in Tabelle 2 aufgeführt.

(Siehe Tabelle 2 Seite 12 f.)

Tabelle 2

| Katalysator Nr. | Katalysator Formel | Bestrahlungs-zeit (Sek.) | Pendelhärte (Sek.) nach 30 Min. | Pendelhärte (Sek.) nach 24 Std. | Farbtonabstand Δ E |
|---|---|---|---|---|---|
| I | 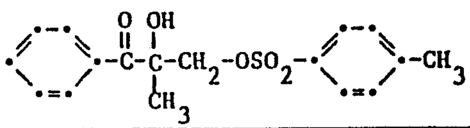 | 2,1<br>4,2<br>8,4<br>12,6 | 169<br>182<br>185<br>186 | 180<br>192<br>193<br>197 | 0,8<br>0,9<br>1,0<br>1,2 |
| II | 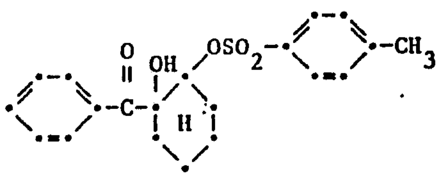 | 2,1<br>4,2<br>8,4<br>12,6 | 150<br>169<br>176<br>176 | 153<br>186<br>190<br>192 | 0,2<br>0,4<br>1,0<br>1,3 |
| III | 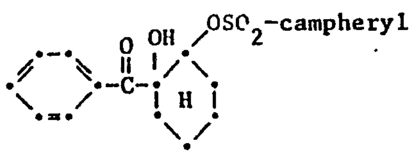 | 2,1<br>4,2<br>8,4<br>12,6 | 76<br>120<br>158<br>169 | 76<br>120<br>169<br>179 | 0,1<br>0,2<br>0,7<br>0,9 |
| IV | 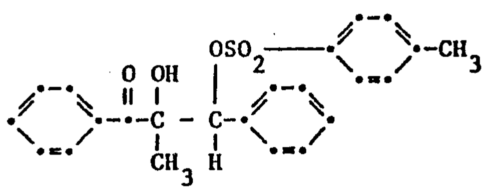 | 2,1<br>4,2<br>8,4<br>12,6 | 158<br>182<br>192<br>195 | 163<br>195<br>200<br>202 | 0,3<br>0,6<br>0,9<br>1,2 |

Ausserdem wird die Lagerstabilität der Lackproben durch Messung der Viskosität mit dem ICI Kegel-Platte-Viskosimeter (DIN 53229) während 7-tägiger Lagerung bei 60 °C überprüft. Bei dieser Methode wird die Viskosität in Poise gemessen.

Tabelle 3

| Katalysator Nr. | Viskosität in Pa . s nach x Tagen Lagerung bei 60 °C | | | | | |
|---|---|---|---|---|---|---|
| | x = 0 | 1 | 2 | 3 | 4 | 7 Tagen |
| I* | 0.28 | 0.32 | 0.36 | 0.38 | 0.42 | 0.5 |
| II* | 0.29 | 0.32 | 0.37 | 0.42 | 0.45 | |
| III* | 0.29 | 0.33 | 0.37 | 0.4 | 0.42 | |
| IV* | 0.32 | 0.42 | 0.6 | 0.88 | 1.68 | geliert |

* Die entsprechenden Formeln der Katalysatoren Nr. I, II, III und IV sind in der Tabelle 2 angegeben.

Beispiel 12

Weitere Lackfilme werden gemäss Beispiel II hergestellt, ihre Pendelhärte gemäss DIN 53 157 zur Beurteilung des Härtungsgrades, und der Farbtonabstand ΔE gemäss DIN 6174 zur Beurteilung der Verfärbung/Vergilbung bestimmt. Die Resultate sind in Tabelle 4 aufgeführt.

(Siehe Tabelle 4 Seite 14 f.)

Tabelle 4

| Nr. | Katalysator Formel | Bestrahlungszeit (Sek.) | Pendelhärte (Sek.) nach 30 Min. | Pendelhärte (Sek.) nach 24 Std. | Farbtonabstand Δ E |
|---|---|---|---|---|---|
| 3 | 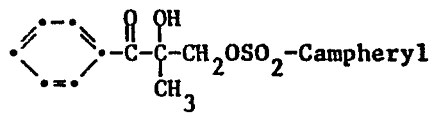 | 0<br>2,1<br>4,2<br>8,4<br>12,6 | 8<br>109<br>153<br>170<br>174 | 8<br>118<br>171<br>192<br>199 | 0,4<br>0,1<br>0,2<br>0,8<br>0,9 |
| 4 | 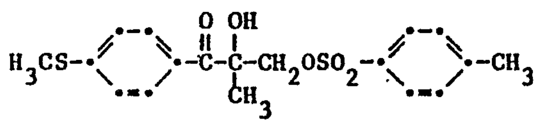 | 0<br>2,1<br>4,2<br>8,4<br>12,6 | 15<br>31<br>47<br>82<br>123 | 14<br>33<br>49<br>82<br>124 | 0,3<br>0,5<br>0,6<br>1,1<br>1,6 |
| 5 | 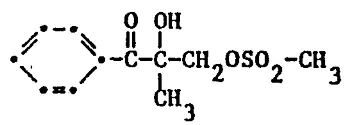 | 0<br>2,1<br>4,2<br>8,4<br>12,6 | 20<br>178<br>188<br>185<br>186 | 18<br>183<br>195<br>197<br>196 | 0,2<br>0,2<br>0,4<br>0,4<br>1,1 |
| 7 | 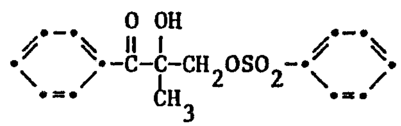 | 0<br>2,1<br>4,2<br>8,4<br>12,6 | 17<br>161<br>180<br>183<br>182 | 12<br>163<br>185<br>191<br>193 | 0,2<br>0,2<br>0,4<br>0,6<br>0,8 |

Ausserdem wird die Lagerstabilität der Lackproben durch Messung der Viskosität mit dem ICI-Kegel-Platte-Viskosimeter (DIN 53 229) während 7-tägiger Lagerung bei 60 °C überprüft. Bei dieser Methode wird die Viskosität in Poise gemessen.

Tabelle 5

| Katalysator Nr. | Viskosität in Pa . s nach x Tagen Lagerung bei 60 °C | | | | | |
|---|---|---|---|---|---|---|
| | x = 0 | 1 | 2 | 3 | 4 | 7 Tagen |
| 3 | 0.3 | 0.35 | 0.38 | 0.40 | 0.42 | 0.53 |
| 4 | 0.3 | 0.35 | 0.4 | 0.42 | 0.44 | 0.49 |
| 5 | 0.3 | 0.35 | 0.39 | 0.42 | 0.46 | 0.5 |
| 7 | 0.31 | 0.36 | 0.38 | 0.44 | 0.47 | 0.55 |

**Patentansprüche**

1. Härtbare Zusammensetzung, enthaltend ein säurehärtbares Harz und als Härtungskatalysator eine Verbindung der Formel I

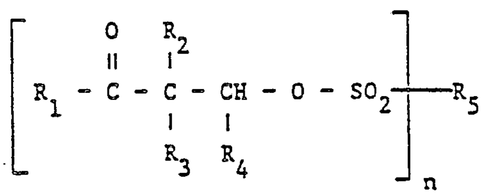

(I)

worin

n die Zahl 1 oder 2 ist, und

$R_1$ ein unsubstituiertes oder durch 1, 2 oder 3 Reste -Cl, -Br, $C_1$-$C_8$ Alkyl, Phenyl, $C_1$-$C_8$ Alkoxy, Phenyloxy, Benzyloxy, $C_1$-$C_8$ Alkylthio, Phenylthio, $-SCH_2CH_2OH$, $C_1$-$C_4$ Alkyl-CONH-, Benzoylamino, Dimethylamino oder durch Benzoyl substituiertes Phenyl oder Naphthyl, oder $R_1$ ferner Anthryl oder Phenanthryl ist,

$R_2$ Wasserstoff, -OH, $C_1$-$C_4$ Alkoxy, $-OSi(CH_3)_3$, $-OCOCH_3$ oder unsubstituiertes oder durch Phenyl substituiertes $C_1$-$C_8$ Alkyl,

$R_3$ Wasserstoff, unsubstituiertes oder durch Phenyl substituiertes $C_1$-$C_8$ Alkyl, -CN, Benzoyl, $C_1$-$C_4$ Alkylcarbonyl oder $C_2$-$C_5$ Alkoxycarbonyl,

$R_4$ Wasserstoff, unsubstuiertes oder durch -OH, -Cl oder Phenyl substituiertes $C_1$-$C_8$ Alkyl, unsubstituiertes oder durch -OH, -Cl, $C_1$-$C_4$ Alkyl oder $C_1$-$C_4$ Alkoxy substituiertes Phenyl, $C_2$-$C_6$ Alkenyl, $C_8$-$C_9$ Phenylalkenyl, Furyl, Thienyl, $-CCl_3$, oder gesättigtes oder ungesättigtes $C_5$-$C_6$ Cycloalkyl bedeuten, oder ferner $R_1$ mit $R_3$, $R_3$ mit $R_4$ oder $R_2$ mit $R_3$ zusammen mit dem Kohlenstoffgerüst, an das sie gebunden sind, einen 5- oder 6-Ring bilden, welcher 1 bis 5 $-CH_2-$, $-CH(CH_3)-$, $-C(CH_3)_2-$, -O-, -S-, -SO-, $-SO_2-$, -CO-, -N(CO-$C_1$-$C_4$ Alkyl)- oder $-N(COC_6H_5)-$Gruppen enthält, und

$R_5$ wenn n = 1, $C_1$-$C_{18}$ Alkyl, unsubstituiertes oder durch -Cl, -OH, $C_1$-$C_{12}$ Alkyl, $C_1$-$C_4$ Alkoxy, $C_1$-$C_4$ Alkyl-CONH-, Benzoylamino, $-NO_2$ oder Benzoyl substituiertes Phenyl, unsubstituiertes oder durch -Cl, $C_1$-$C_{12}$ Alkyl oder $C_1$-$C_4$ Alkoxy substituiertes Naphthyl, oder $R_5$ ferner $C_5$-$C_6$ Cycloalkyl, $C_7$-$C_9$ Aralkyl, Campheryl, $-CF_3$, $CCl_3$, -F oder $-NH_2$ bedeutet, und

$R_5$ wenn n = 2, eine $-(CH_2)_m-$Gruppe, wobei m die Zahl 2 bis 8 darstellt, oder unsubstituiertes oder durch $C_1$-$C_{12}$ Alkyl substituiertes Phenylen oder Naphthylen ist.

2. Zusammensetzung gemäss Anspruch 1, worin der Härtungskatalysator eine Verbindung der Formel I ist, worin n die Zahl 1 oder 2 ist,

$R_1$ ein unsubstituiertes oder durch -Cl, $C_1$-$C_8$ Alkyl, Phenyl, $C_1$-$C_4$ Alkoxy, Phenyloxy, $C_1$-$C_4$ Alkylthio, oder durch $-SCH_2CH_2OH$ substituiertes Phenyl oder Naphthyl ist,

$R_2$ Wasserstoff, -OH oder $C_1$-$C_8$ Alkyl,

$R_3$ Wasserstoff oder $C_1$-$C_8$ Alkyl, und

$R_4$ Wasserstoff, $C_1$-$C_4$ Alkyl, Phenyl, $C_2$-$C_6$ Alkenyl, Furyl oder $-CCl_3$ bedeuten, oder ferner $R_2$ mit $R_3$ oder $R_3$ mit $R_4$ zusammen mit den Kohlenstoffatomen, an die sie gebunden sind, einen Cyclohexylring bilden, und

$R_5$ wenn n = 1, $C_1$-$C_{18}$ Alkyl, unsubstituiertes oder durch -Cl, $C_1$-$C_{12}$ Alkyl oder $C_1$-$C_4$ Alkoxy substituiertes Phenyl, unsubstituiertes oder durch $C_1$-$C_{12}$ Alkyl substituiertes Naphthyl, oder $R_5$ ferner Campheryl, $-CF_3$ oder -F bedeutet, und

$R_5$ wenn n = 2, eine $-(CH_2)_m-$Gruppe, Phenylen oder Naphthylen ist, worin m die Zahl 2, 3 oder 4 darstellt.

3. Zusammensetzung gemäss Anspruch 1, worin der Härtungskatalysator eine Verbindung der

Formel I ist, worin n die Zahl 1 ist.

4. Zusammensetzung gemäss Anspruch 1, worin der Härtungskatalysator eine Verbindung der Formel I ist, worin n die Zahl 1 ist,

$R_1$ ein unsubstituiertes oder durch -Cl, $C_1$-$C_4$ Alkyl, $C_1$-$C_4$ Alkoxy, $-SCH_3$ oder Phenyl substituiertes Phenyl ist,

$R_2$ -OH oder $C_1$-$C_4$ Alkyl, $R^3$ $C_1$-$C_4$ Alkyl und $R_4$ Wasserstoff, $C_1$-$C_4$ Alkyl, Furyl oder $-CCl_3$ bedeuten, oder ferner $R_3$ mit $R_4$ zusammen mit den Kohlenstoffatomen, an die sie gebunden sind, einen Cyclohexylring bilden, und

$R_5$ $C_1$-$C_{18}$ Alkyl, unsubstituiertes oder durch $C_1$-$C_{12}$ Alkyl substituiertes Phenyl oder Naphthyl, oder Campheryl bedeutet.

5. Zusammensetzung gemäss Anspruch 1, worin der Härtungskatalysator eine Verbindung der Formel I ist, worin $R_2$ -OH und $R_3$ $C_1$-$C_4$ Alkyl bedeuten.

6. Zusammensetzung gemäss Anspruch 1, worin der Härtungskatalysator eine Verbindung der Formel I ist, worin $R_3$ mit $R_4$ zusammen mit den Kohlenstoffatomen, an die sie gebunden sind, einen Cyclohexylring bilden.

7. Zusammensetzung gemäss Anspruch 1, worin der Härtungskatalysator eine Verbindung der Formel I ist, worin $R_5$ ein unsubstituiertes oder durch $C_1$-$C_{12}$ Alkyl substituiertes Phenyl ist.

8. Zusammensetzung gemäss Anspruch 1, worin der Härtungskatalysator eine Verbindung der Formel I ist, worin $R_1$ Phenyl, $R_2$ -OH, $R_3$ $-CH_3$, $R_4$ -H und $R_5$ Phenyl, p-Tolyl oder p-n-Dodecylphenyl bedeuten.

9. Zusammensetzung gemäss Anspruch 1, worin der Härtungskatalysator eine Verbindung der Formel I ist, worin $R_1$ Phenyl, $R_2$ -OH, $R_3$ mit $R_4$ zusammen mit dem Kohlenstoffgerüst, an das sie gebunden sind, einen Cyclohexylring bilden, und $R_5$ p-Tolyl bedeuten.

10. Verbindungen der Formel X

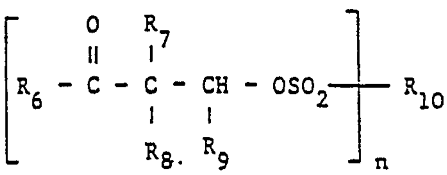

(X)

worin n die Zahl 1 oder 2 ist,

$R_6$ ein unsubstituiertes oder durch, 1, 2 oder 3 Reste -Cl, -Br, $C_1$-$C_8$ Alkyl, Phenyl, $C_1$-$C_8$ Alkoxy, Phenyloxy, Benzyloxy, $C_1$-$C_8$ Alkylthio, Phenylthio, $-SCH_2CH_2OH$, $C_1$-$C_4$ Alkyl-CONH-, Benzoylamino, Dimethylamino oder Benzoyl substituiertes Phenyl oder Naphthyl, oder $R_6$ ferner Anthryl oder Phenanthryl ist,

$R_7$ -H, -OH, $C_1$-$C_4$ Alkoxy, $-OSi(CH_3)_3$, $-OCOCH_3$, $C_2$-$C_8$ Alkyl oder durch Phenyl substituiertes $C_1$-$C_8$ Alkyl,

$R_8$ unsubstituiertes oder durch Phenyl substituiertes $C_1$-$C_8$ Alkyl, -CN Benzoyl, $C_1$-$C_4$ Alkylcarbonyl oder $C_2$-$C_5$ Alkoxycarbonyl, und

$R_9$ -H, unsubstituiertes oder durch -OH, -Cl oder Phenyl substituiertes $C_1$-$C_8$ Alkyl, unsubstituiertes oder durch -OH, -Cl, $C_1$-$C_4$ Alkyl oder $C_1$-$C_4$ Alkoxy substituiertes Phenyl, $C_2$-$C_6$ Alkenyl, $C_8$-$C_9$ Phenylalkenyl, Furyl, Thienyl, $-CCl_3$, oder gesättigtes oder ungesättigtes $C_5$-$C_6$ Cycloalkyl bedeuten, oder ferner $R_6$ mit $R_8$, $R_8$ mit $R_9$ oder $R_7$ mit $R_8$ zusammen mit dem Kolhenstoffgerüst, an das sie gebunden sind, einen 5- oder 6-Ring bilden, welcher 1 bis 5 $-CH_2-$, $-CH(CH_3)-$, $-C(CH_3)_2-$, -O-, -S-, $-SO_2-$, -CO-, -N(CO-$C_1$-$C_4$ Alkyl)-, oder $-N(COC_6H_5)$-Gruppen enthält, und

$R_{10}$ wenn n = 1, $C_1$-$C_{18}$ Alkyl, unsubstituiertes oder durch -Cl, -OH, $C_1$-$C_{12}$ Alkyl, $C_1$-$C_4$ Alkoxy, $C_1$-$C_4$ Alkyl-CONH-, Benzoylamino, $-NO_2$ oder durch Benzoyl substituiertes Phenyl, unsubstituiertes oder durch -Cl, $C_1$-$C_{12}$ Alkyl oder $C_1$-$C_4$ Alkoxy substituiertes Naphthyl, oder $R_{10}$ ferner $C_5$-$C_6$ Cycloalkyl, $C_7$-$C_9$ Aralkyl, Campheryl, $-CF_3$, $-CCl_3$, -F oder $-NH_2$ bedeutet, und

$R_{10}$ wenn n = 2, eine $-(CH_2)_m$-Gruppe oder durch $C_1$-$C_{12}$ Alkyl substituiertes Phenylen oder Naphthylen ist.

11. Zusammensetzung gemäss Anspruch 1, dadurch gekennzeichnet, dass die härtbare Zusammensetzung 0,1 bis 10 Gew.%, bezogen auf das lösungsmittelfreie Harz, eines Härtungskatalysators der Formel I enthält.

12. Zusammensetzung gemäss Anspruch 1, dadurch gekennzeichnet, dass sie ausser dem Harz und dem Härtungskatalysator noch sonstige, in der Technologie der Harze übliche Zusatzstoffe enthält.

13. Zusammensetzung gemäss Anspruch 1, dadurch gekennzeichnet, dass das säurehärtbare Harz ein Gemisch aus mindestens einer polymerisierbaren Verbindung mit einer oder mehreren polymerisierbaren, äthylenisch ungesättigten Bindungen und zusätzlich mindestens einem Aminoplast ist.

14. Zusammensetzung gemäss Anspruch 1, dadurch gekennzeichnet, dass das säurehärtbare Harz ein Phenolharz, ein Phenol-Formaldehydharz, ein Harnstoff-Formaldehydharz oder ein Gemisch eines solchen Harzes mit einem anderen säurehärtbaren Harz ist.

15. Zusammensetzung gemäss Anspruch 1, dadurch gekennzeichnet, dass das säurehärtbare Harz

ein Gemisch von polyfunktionellen Alkoholen, oder Hydroxylgruppen enthaltenden Acryl- oder Polyesterharzen, oder partiell verseiftem Polyvinylacetat oder Polyvinylalkohol mit polyfunktionellen Dihydropyranyläthern ist.

16. Verfahren zum Härten von säurehärtbaren Harzen, die einen Härtungskatalysator gemäss Anspruch 1 enthalten, dadurch gekennzeichnet, dass man das Harz mit kurzwelligem Licht bestrahlt und anschliessend erwärmt.

17. Verwendung einer Zusammensetzung gemäss Anspruch 1 zur industriellen Beschichtung und Lackierung.

18. Verwendung einer Zusammensetzung gemäss Anspruch 1 zum Blechdruck.

19. Verwendung einer Zusammensetzung gemäss Anspruch 1 zur Herstellung einer Relief-Form.

**Claims**

1. A curable composition containing an acid-curable resin and, as the curing catalyst, a compound of the formula I

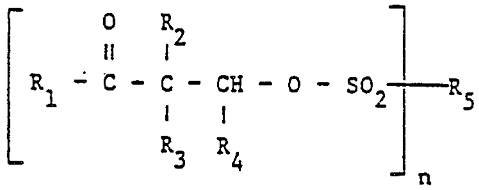

(I)

in which

n is the number 1 or 2, and

$R_1$ is unsubstituted phenyl or naphthyl, or phenyl or naphthyl which is substituted by 1, 2 or 3 radicals from the group comprising —Cl, —Br, $C_1$-$C_8$-alkyl, phenyl, $C_1$-$C_8$-alkoxy, phenoxy, benzyloxy, $C_1$-$C_8$-alkylthio, phenylthio, $—SCH_2CH_2OH$, $C_1$-$C_4$-alkyl-CONH-, benzoylamino and dimethylamino or by benzoyl, or $R_1$ furthermore is anthryl or phenanthryl,

$R_2$ is hydrogen, —OH, $C_1$-$C_4$-alkoxy, $—OSi(CH_3)_3$ or $—OCOCH_3$, or is $C_1$-$C_8$-alkyl which is unsubstituted or substituted by phenyl

$R_3$ is hydrogen, unsubstituted or phenyl-substituted $C_1$-$C_4$-alkyl, —CN, benzoyl, $C_1$-$C_8$-alkylcarbonyl, or $C_2$-$C_5$-alkoxycarbonyl,

$R_4$ is hydrogen, $C_1$-$C_8$-alkyl which is unsubstituted or substituted by —OH, —Cl or phenyl, phenyl which is unsubstituted or substituted by —OH, —Cl, $C_1$-$C_4$-alkyl or $C_1$-$C_4$-alkoxy, $C_2$-$C_6$-alkenyl, $C_8$-$C_9$-phenylalkenyl, furyl, thienyl, $—CCl_3$ or saturated or unsaturated $C_5$-$C_6$-cycloalkyl, or, furthermore, $R_1$ and $R_3$, $R_3$ and $R_4$ or $R_2$ and $R_3$, together with the carbon skeleton to which they are bonded, form a 5-membered or 6-membered ring which contains 1 to 5 $—CH_2—$, $—CH(CH_3)—$, $—C(CH_3)_2—$, —O—, —S—, —SO—, $—SO_2—$, —CO—, —N(CO-$C_1$-$C_4$-alkyl)— or $—N(COC_6H_5)$-groups, and,

if n is 1, $R_5$ is $C_1$-$C_{18}$-alkyl, phenyl which is unsubstituted or substituted by —Cl, —OH, $C_1$-$C_{12}$-alkyl, $C_1$-$C_4$-alkoxy, $C_1$-$C_4$-alkyl-CONH-, benzoylamino, $—NO_2$ or benzoyl, or naphthyl which is unsubstituted or substituted by —Cl, $C_1$-$C_{12}$-alkyl or $C_1$-$C_4$-alkoxy, or $R_5$ furthermore is $C_5$-$C_6$-cycloalkyl, $C_7$-$C_9$-aralkyl, camphoryl, $—CF_3$, $—CCl_3$, —F or $—NH_2$, or,

if n is 2, $R_5$ is a $—(CH_2)_m$-group, in which m is a number from 2 to 8, or unsubstituted or $C_1$-$C_{12}$-alkyl-substituted phenylene or naphtylene.

2. A composition according to claim 1, wherein the curing catalyst is a compound of the formula I in which n is the number 1 or 2,

$R_1$ is unsubstituted phenyl or naphthyl, or phenyl or naphthyl which is substituted by —Cl, $C_1$-$C_8$-alkyl, phenyl, $C_1$-$C_4$-alkoxy, phenoxy or $C_1$-$C_4$-alkylthio or by $—SCH_2CH_2OH$,

$R_2$ is hydrogen, —OH or $C_1$-$C_8$-alkyl,

$R_3$ is hydrogen or $C_1$-$C_8$-alkyl and

$R_4$ is hydrogen, $C_1$-$C_4$-alkyl, phenyl, $C_2$-$C_6$-alkenyl, furyl or $—CCl_3$, or, furthermore, $R_2$ and $R_3$, or $R_3$ and $R_4$, together with the carbon atoms to which they are bonded, form a cyclohexyl ring, and,

if n is 1, $R_5$ is $C_1$-$C_{18}$-alkyl, phenyl which is unsubstituted or substituted by —Cl, $C_1$-$C_{12}$-alkyl or $C_1$-$C_4$-alkoxy, or naphthyl which is unsubstituted or substituted by $C_1$-$C_{12}$-alkyl, or $R_5$ furthermore is camphoryl, $—CF_3$ or —F, or,

if n is 2, $R_5$ is a $—(CH_2)_m$ group, phenylene or naphthylene, in which m is the number 2, 3 or 4.

3. A composition according to claim 1, wherein the curing catalyst is a compound of the formula I in which n is the number 1.

4. A composition according to claim 1, wherein the curing catalyst is a compound of the formula I in which n is the number 1,

$R_1$ is phenyl which is unsubstituted or substituted by —Cl, $C_1$-$C_4$-alkyl, $C_1$-$C_4$-alkoxy, $—SCH_3$ or phenyl,

$R_2$ is —OH or $C_1$-$C_4$-alkyl, $R_3$ is $C_1$-$C_4$-alkyl and $R_4$ is hydrogen, $C_1$-$C_4$-alkyl, furyl or $—CCl_3$, or,

furthermore, $R_3$ and $R_4$, together with the carbon atoms to which they are bonded, form a cyclohexyl ring, and

$R_5$ is $C_1$-$C_{18}$-alkyl, unsubstituted or $C_1$-$C_{12}$-alkyl-substituted phenyl or naphthyl, or camphoryl.

5. A composition according to claim 1, wherein the curing catalyst is a compound of the formula I in which $R_2$ is —OH and $R_3$ is $C_1$-$C_4$-alkyl.

6. A composition according to claim 1, wherein the curing catalyst is a compound of the formula I in which $R_3$ and $R_4$, together with the carbon atoms to which they are bonded, form a cyclohexyl ring.

7. A composition according to claim 1, wherein the curing catalyst is a compound of the formula I in which $R_5$ is unsubstituted or ($C_1$-$C_{12}$-alkyl) substituted phenyl.

8. A composition according to claim 1, wherein the curing catalyst is a compound of the formula I in which $R_1$ is phenyl, $R_2$ is —OH, $R_3$ is $—CH_3$, $R_4$ is —H and $R_5$ is phenyl, p-tolyl or p-n-dodecylphenyl.

9. A composition according to claim 1, wherein the curing catalyst is a compound of the formula I in which $R_1$ is phenyl, $R_2$ is —OH, $R_3$ and $R_4$, together with the carbon skeleton to which they are bonded, form a cyclohexyl ring and $R_5$ is p-tolyl.

10. A compound of the formula X

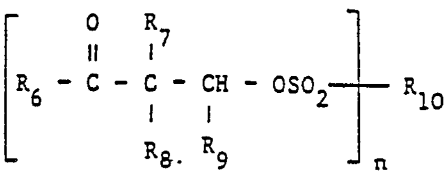

(X)

in which n is the number 1 or 2,

$R_6$ is unsubstituted phenyl or naphthyl, or phenyl or naphthyl which is substituted by 1, 2 or 3 radicals from the group comprising —Cl, —Br, $C_1$-$C_8$-alkyl, phenyl, $C_1$-$C_8$-alkoxy, phenoxy, benzyloxy, $C_1$-$C_8$-alkylthio, phenylthio, $—SCH_2CH_2OH$, $C_1$-$C_4$-alkyl-CONH-, benzoylamino, dimethylamino or benzoyl, or $R_6$ furthermore is anthryl or phenanthryl,

$R_7$ is —H, —OH, $C_1$-$C_4$-alkoxy, $—OSi(CH_3)_3$, $—OCOCH_3$, $C_2$-$C_8$-alkyl, or $C_1$-$C_8$-alkyl which is substituted by phenyl,

$R_8$ is unsubstituted or phenyl-substituted $C_1$-$C_8$-alkyl, —CN, benzoyl, $C_1$-$C_4$-alkylcarbonyl or $C_2$-$C_5$-alkoxycarbonyl, and

$R_9$ is —H, $C_1$-$C_8$-alkyl which is unsubstituted or substituted by —OH, —Cl or phenyl, phenyl which is unsubstituted or substituted by —OH, —Cl, $C_1$-$C_4$-alkyl or $C_1$-$C_4$-alkoxy, $C_2$-$C_6$-alkenyl, $C_8$-$C_9$-phenylalkenyl, furyl, thienyl, $—CCl_3$ or saturated or unsaturated $C_5$-$C_6$-cycloalkyl, or furthermore $R_6$ and $R_8$, $R_8$ and $R_9$ or $R_7$ and $R_8$, together with the carbon skeleton to which they are bonded, form a 5-membered or 6-membered ring which contains 1 to 5 $—CH_2—$, $—CH(CH_3)—$, $—C(CH_3)_2—$, —O—, —S—, $—SO_2—$, —CO—, —N(CO-$C_1$-$C_4$-alkyl)— or $—N(COC_6H_5)$- groups, and, if

n is 1, $R_{10}$ is $C_1$-$C_{18}$-alkyl, phenyl which is unsubstituted or sustituted by —Cl, —OH, $C_1$-$C_{12}$-alkyl, $C_1$-$C_4$-alkoxy, $C_1$-$C_4$-alkyl-CONH-, benzoylamino, $—NO_2$ or by benzoyl, naphthyl which is unsubstituted or substituted by —Cl, $C_1$-$C_{12}$-alkyl or $C_1$-$C_4$-alkoxy, or $R_{10}$ furthermore is $C_5$-$C_6$-cycloalkyl, $C_7$-$C_9$-aralkyl, camphoryl, $—CF_3$, $—CCl_3$, —F or $—NH_2$, or

if n is 2, $R_{10}$ is a $—(CH_2)_m$-group or $C_1$-$C_{12}$-alkyl-substituted phenylene or naphthylene.

11. A composition according to claim 1, which curable composition contains 0,1 to 10 % by weight, based on the solvent-free resin, of a curing catalyst of the formula I.

12. A composition according to claim 1, which also contains, in addition to the resin and the curing catalyst, other additives which are conventional in the technology of resins.

13. A composition according to claim 1, wherein the acid-curable resin is a mixture of a least one polymerisable compound with one or more polymerisable ethylenically unsaturated bonds and, in addition, at least one aminoplast.

14. A composition according to claim 1, wherein the acid-curable resin is a phenolic resin, a phenol/formaldehyde resin, a urea/formaldehyde resin or a mixture of such a resin and another acid-curable resin.

15. A composition according to claim 1, wherein the acid-curable resin is a mixture of polyfunctional alcohols, or acrylic or polyester resins containing hydroxyl groups, or partially hydrolysed polyvinyl acetate or polyvinyl alcohol with polyfunctional dihydropyranyl ethers.

16. A process for curing acid-curable resins containing a curing catalyst according to claim 1, which comprises irradiating the resin with light of short wavelength and then warming it.

17. The use of a composition according to claim 1 for industrial coating and varnisching.

18. The use of a composition according to claim 1 for tinplate printing.

19. The use of a composition according to claim 1 for the production of a relief mould.

**Revendications**

1. Composition durcissable qui contient une résine durcissable par un acide et, comme catalyseur de durcissement, un composé répondant à la formule I :

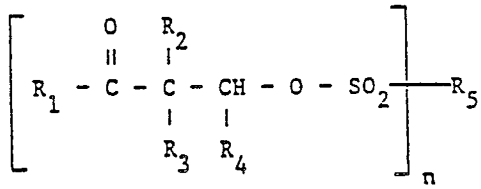

(I)

dans laquelle :

n désigne le nombre 1 ou le nombre 2,

$R_1$ représente un radical phényle ou naphtyle non substitué ou porteur d'1, 2 ou 3 substituants pris dans l'ensemble constitué par Cl, Br, les alkyles en $C_1$-$C_8$, le phényle, les alcoxy en $C_1$-$C_8$, le phényloxy, le benzyloxy, les alkylthio en $C_1$-$C_8$, le phénylthio, $—SCH_2CH_2OH$, les radicaux alkylcarbonylamino à alkyle en $C_1$-$C_4$, le benzoylamino et le diméthylamino, ou porteur d'un radical benzoyle, ou encore $R_1$ représente un radical anthryle ou phénanthryle,

$R_2$ représente l'hydrogène, —OH, un alcoxy en $C_1$-$C_4$, $—OSi(CH_3)_3$, $—OCOCH_3$ ou un radical alkyle en $C_1$-$C_8$ non substitué ou porteur d'un phényle,

$R_3$ représente l'hydrogène, un alkyle en $C_1$-$C_8$ non substitué ou porteur d'un phényle, ou représente —CN, un benzoyle, un alkylcarbonyle à alkyle en $C_1$-$C_4$ ou un alcoxycarbonyle en $C_2$-$C_5$,

$R_4$ représente l'hydrogène, un alkyle en $C_1$-$C_8$ non substitué ou porteur d'un —OH, d'un —Cl ou d'un phényle, un phényle non substitué ou porteur d'un —OH, d'un Cl, d'un alkyle en $C_1$-$C_4$ ou d'un alcoxy en $C_1$-$C_4$, un alcényle en $C_2$-$C_6$, un phénylalcényle en $C_8$ ou $C_9$, un furyle, un thiényle, un $—CCl_3$ ou un cycloalkyle en $C_5$ ou $C_6$, saturé ou insaturé, ou encore les deux radicaux d'un des couples ($R_1$, $R_3$), ($R_3$, $R_4$) et ($R_2$, $R_3$) forment ensemble, et avec le squelette carboné auquel ils sont liés, un cycle pentagonal ou hexagonal qui contient de 1 à 5 des radicaux $—OH_2—$, $—OH(CH_3)—$, $—C(CH_3)_2—$, —O—, —S—, —SO—, $—SO_2—$, —CO—, —N(CO-$C_1$-$C_4$-alkyl)— et $—N(COC_6H_5)$, et

$R_5$ représente :

— lorsque n est égal à 1, un alkyle en $C_1$-$C_{18}$, un phényle non substitué ou porteur d'un —Cl, d'un —OH, d'un alkyle en $C_1$-$C_{12}$, d'un alcoxy en $C_1$-$C_4$, d'un radical alkyl-CONH- à alkyle en $C_1$-$C_4$, d'un benzoylamino, d'un $—NO_2$ ou d'un benzoyle, un naphtyle non substitué ou porteur d'un —Cl, d'un alkyle en $C_1$-$C_{12}$ ou d'un alcoxy en $C_1$-$C_4$, ou encore $R_5$ représente un cycloalkyle en $C_5$ ou $C_6$, un aralkyle en $C_7$-$C_9$, un camphéryle, un $—CF_3$, un $—CCl_3$, un —F ou un $—NH_2$, et

— lorsque n est égal à 2, un radical $—(CH_2)_m—$ dans lequel m désigne un nombre 2 à huit, ou un radical phénylène ou naphtylène non substitué ou porteur d'un alkyle en $C_1$-$C_{12}$.

2. Composition selon la revendication 1 qui contient, comme catalyseur de durcissement, un composé de formule I dans lequel :

n représente le nombre 1 ou le nombre 2,

$R_1$ représente un radical phényle ou naphtyle non substitué ou porteur d'un substituant pris dans l'ensemble constitué par —Cl, les alkyles en $C_1$-$C_8$, le phényle, les alcoxy en $C_1$-$C_4$, le phényloxy, les alkylthio en $C_1$-$C_4$ et le radical $—SCH_2CH_2OH$,

$R_2$ représente l'hydrogène, —OH ou un alkyle en $C_1$-$C_8$,

$R_3$ représente l'hydrogène ou un alkyle en $C_1$-$C_8$,

$R_4$ représente l'hydrogène, un alkyle en $C_1$-$C_4$, un phényle, un alcényle en $C_2$-$C_6$, un furyle ou $—CCl_3$, ou encore le couple ($R_2$, $R_3$) ou le couple ($R_3$, $R_4$) forme, avec le ou les atomes de carbone qui le porte(nt), un noyau de cyclohexane, et

$R_5$ représente :

— dans le cadre où n est égal à 1, un alkyle en $C_1$-$C_{18}$, un phényle non substitué ou porteur d'un —Cl, d'un alkyle en $C_1$-$C_{12}$ ou d'un alcoxy en $C_1$-$C_4$, un naphtyle non substitué ou porteur d'un alkyle en $C_1$-$C_{12}$, un camphéryle, $—CF_3$ ou —F, et

— dans le cas où n est égal à 2, un radical $—(CH_2)_m—$ dont l'indice m est étal à 2, à 3 ou à 4, un phénylène ou un naphtylène.

3. Composition selon la revendication 1 qui contient, comme catalyseur de durcissement, un composé de formule I dans lequel n est égal à 1.

4. Composition selon la revendication 1 qui contient, comme catalyseur de durcissement, un composé de formule I dans lequel :

n représente le nombre 1,

$R_1$ représente un radical phényle non substitué ou porteur d'un —Cl, d'un alkyle en $C_1$-$C_4$, d'un alcoxy en $C_1$-$C_4$, d'un radical $—SCH_3$ ou d'un radical phényle,

$R_2$ représente —OH ou un alkyle en $C_1$-$C_4$,

$R_3$ représente un alkyle en $C_1$-$C_4$,

$R_4$ représente l'hydrogène, un alkyle en $C_1$-$C_4$, un furyle ou un $—CCl_3$, ou encore $R_3$ et $R_4$ forment ensemble, et avec les atomes de carbone auxquels ils sont liés, un radical de cyclohexane, et

$R_5$ représente un alkyle en $C_1$-$C_{18}$, un radical phényle ou naphtyle non substitué ou porteur d'un

alkyle en $C_1$-$C_{12}$, ou un radical camphéryle.

5. Composition selon la revendication 1 qui contient, comme catalyseur de durcissement, un composé de formule I dans lequel $R_2$ représente -OH et $R_3$ représente un alkyle en $C_1$-$C_4$.

6. Composition selon la revendication 1 qui contient, comme catalyseur de durcissement, un composé de formule I dans lequel $R_3$ et $R_4$ forment ensemble, et avec les atomes de carbone auxquels ils sont liés, un noyau de cyclohexane.

7. Composition selon la revendication 1 qui contient, comme catalyseur de durcissement, un composé de formule I dans lequel $R_5$ représente un radical phényle non substitué ou porteur d'un alkyle en $C_1$-$C_{12}$.

8. Composition selon la revendication 1 qui contient, comme catalyseur de durcissement, un composé de formule I dans lequel $R_1$ représente un phényle, $R_2$ un -OH, $R_3$ un $-CH_3$, $R_4$ un -H et $R_5$ un radical phényle, p-totyle ou n-dodécyl-4 phényle.

9. Composition selon la revendication 1 qui contient, comme catalyseur de durcissement, un composé de formule I dans lequel $R_1$ représente un phényle, $R_2$ un -OH, $R_3$ et $R_4$ forment ensemble, et avec le squelette carboné auquel ils sont liés, un radical de cyclohexane et $R_5$ représente un radical p-totyle.

10. Composés qui répondent à la formule X :

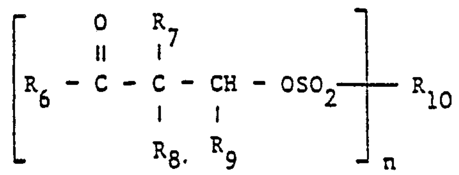

(X)

dans laquelle :

n représente le nombre 1 ou le nombre 2,

$R_6$ représente un radical phényle ou naphtyle non substitué ou porteur d'1, de 2 ou de 3 substituants pris dans l'ensemble constitué par -Cl, -Br, les alkyles en $C_1$-$C_8$, le phényle, les alcoxy en $C_1$-$C_8$, le phényloxy, le benzyloxy, les alkylthio en $C_1$-$C_8$, le phénylthio, $-SCH_2CH_2OH$, les alkylcarbonylamino à alkyle en $C_1$-$C_4$, le benzoylamino, le diméthylamino et le benzoyle, ou encore $R_6$ représente un radical anthryle ou phénanthryle,

$R_7$ représente -H, -OH, un alcoxy en $C_1$-$C_4$, $-OSi(CH_3)_3$, $-OCOCH_3$, un alkyle en $C_2$-$C_8$ ou un alkyle en $C_1$-$C_8$ porteur d'un phényle,

$R_8$ représente un alkyle en $C_1$-$C_8$ non substitué ou porteur d'un phényle, un cyano, un benzoyle, un alkylcarbonyle à alkyle en $C_1$-$C_4$ ou un alcoxycarbonyle en $C_2$-$C_5$,

$R_9$ représente -H, un alkyle en $C_1$-$C_8$ non substitué ou porteur d'un -OH, d'un -Cl ou d'un phényle, un phényle non substitué ou porteur d'un -OH, d'un -Cl, d'un alkyle en $C_1$-$C_4$ ou d'un alcoxy en $C_1$-$C_4$, un alcényle en $C_2$-$C_6$, un phénylalcényle en $C_8$ ou $C_9$, un furyle, un thiényle, $-CCl_3$ ou un cycloalkyle en $C_5$ ou $C_6$ saturé ou non, ou encore les radicaux du couple ($R_6$, $R_8$), ($R_8$, $R_9$) ou ($R_7$, $R_8$) forment, avec le squelette carboné auquel ils sont liés, un cycle pentagonal ou hexagonal qui contient de 1 à 5 des radicaux $-CH_2-$, $-CH(CH_3)-$, $-C(CH_3)_2-$, -O-, -S-, $-SO_2-$, -CO-, -N(CO-alkyl)- à alkyle en $C_1$-$C_4$, ou $-N(COC_6H_5)-$, et

$R_{10}$ représente :

— dans le cas où n est égal à 1, un alkyle en $C_1$-$C_{18}$, un phényle non substitué ou porteur d'un -Cl, d'un -OH, d'un alkyle en $C_1$-$C_{12}$, d'un alcoxy en $C_1$-$C_4$, d'un alkylcarbonylamino à alkyle en $C_1$-$C_4$, d'un benzoylamino, d'un $-NO_2$ ou d'un benzoyle, un naphtyle non substitué ou porteur d'un -Cl, d'un alkyle en $C_1$-$C_{12}$ ou d'un alcoxy en $C_1$-$C_4$, un cycloalkyle en $C_5$ ou $C_6$, un aralkyle en $C_7$-$C_9$, un camphéryle, un $-CF_3$, un $-CCl_3$, un -F ou un $-NH_2$, et

— dans le cas où n est égal à 2, un radical $-(CH_2)_m-$ ou un radical phénylène ou naphtylène porteur d'un alkyle en $C_1$-$C_{12}$.

11. Composition selon la revendication 1 caractérisée en ce que la composition durcissable contient de 0,1 à 10 % en poids, par rapport à la résine sans solvant, d'un catalyseur de durcissement de formule I.

12. Composition selon la revendication 1 caractérisée en ce qu'elle contient, en plus de la résine et du catalyseur de durcissement, d'autres additifs pris parmi ceux que l'on utilise ordinairement dans la technique des résines.

13. Composition selon la revendication 1 caractérisée en ce que la résine durcissable par un acide est un mélange d'au moins un composé polymérisable renfermant une ou plusieurs liaisons éthyléniques polymérisables et, en plus, d'au moins un aminoplaste.

14. Composition selon la revendication 1 caractérisée en ce que la résine durcissable par un acide est une résine phénolique, une résine phénol/formaldéhyde, une résine urée/formaldéhyde ou un mélange d'une résine de ce genre avec une autre résine durcissable par un acide.

15. Composition selon la revendication 1 caractérisée en ce que la résine durcissable par un acide est un mélange de polyols, ou de résines acryliques ou polyesters contenant des radicaux hydroxy, ou d'un poly(acétate de vinyle) partiellement saponifié ou d'un poly(alcool vinylique) avec des éthers dihydropyrannyliques polyfonctionnels.

16. Procédé pour durcir des résines durcissables par un acide qui contiennent un catalyseur de durcissement selon la revendication 1, procédé caractérisé en ce qu'on expose la résine à un rayonnement de courte longueur d'onde, puis on la chauffe.

17. Application d'une composition selon la revendication 1 à l'enduction et au peinturage industriels.

18. Application d'une composition selon la revendication 1 à l'impression sur tôles.

19. Application d'une composition selon la revendication 1 à la fabrication d'un moule en relief.